# EUROPÄISCHE PATENTSCHRIFT

(11) **EP 1 338 271 B1**
(45) Veröffentlichungstag und Bekanntmachung des Hinweises auf die Patenterteilung: **15.07.2009**
(21) Anmeldenummer: 02026130.1
(22) Anmeldetag: 23.11.2002
(51) Int. Cl.: A61K 9/107, A23P 1/04, A61K 9/48, A61K 31/375, A61K 31/355, A61K 31/07, A61K 31/20

(54) **Wässrige Lösung von Ascorbinsäure**
Aqeous solution of ascorbic acid
Solution aqueuse d'acide ascorbique

(30) Priorität: 30.11.2001 DE 10158447
(43) Veröffentlichungstag der Anmeldung: 27.08.2003
(60) Teilanmeldung: 06002865.1
(73) Patentinhaber: AQUANOVA AG, 64295 Darmstadt (DE)
(72) Erfinder: Behnam, Dariush, 64380 Rossdorf (DE)
(74) Vertreter: Blumbach - Zinngrebe

(56) Entgegenhaltungen:
- EP-A- 0 352 375
- EP-A- 0 440 398
- EP-A- 0 821 950
- EP-A- 0 956 779
- WO-A-02/43719
- WO-A-99/39580
- GB-A- 780 886
- US-A- 3 249 504
- US-A- 5 607 707
- US-A- 5 656 289
- POUST RI ET AL.: "Copper-catalyzed oxidation of ascorbic acid in gels and aqueous solutions of polysorbate 80" JOURNAL OF PHARMACEUTICAL SCIENCES, Bd. 57, Nr. 12, 1968, Seiten 2119-25, XP009005867

## Beschreibung

Die Erfindung betrifft eine wässrige Lösung von Ascorbinsäure.

Die technische, insbesondere die körperpflegemittel- und lebensmitteltechnische Verwendung und prophylaktische Anwendung des Reduktons Ascorbinsäure wird oft dadurch erschwert, daß die Ascorbinsäure in wässriger Lösung nicht ausreichend beständig ist. So sind in einer wässrigen Ascorbinsäurelösung schon nach etwa 30 Tagen nur noch etwa 70% der zu zugesetzten Ascorbinsäure vorhanden.

Man hat die Beständigkeit dadurch zu verbessern versucht, daß man statt Ascorbinsäure eines ihrer Derivate, beispielsweise Natriumascorbinphosphat oder Ascorbylpalmitat eingesetzt hat. Der Gehalt dieses Wirkstoffes bleibt in wässriger Lösung zwar über lange Zeit nahezu erhalten. Jedoch ist das Derivat um ein Vielfaches teurer als reine Ascorbinsäure. Außerdem neigen die Ascorbinsäurederivate bei höherer Konzentration zum Auskristallisieren und führen zu einer Einfärbung des Endproduktes.

In der europäischen Patentschrift 660676 ist eine Zusammensetzung bestehend aus 0,1-2,0 Gew% eines öllöslichen Bestandteils, der bevorzugt ein farbgebendes Carotenoid ist, aus 2-20 Gew% eines Emulgators mit einem HLB-Wert von 10-18 und aus 0,1-1,0Gew% eines Antoxidationsmittels bekannt. Der Emulgator kann Polysorbat 40 oder 60 sein, als Antioxidationsmittel wird unter anderem Ascorbinsäure empfohlen. Damit sollen farblich klare und stabile Getränke für die Gesundheitsvorsorge herstellbar sein. Ungeklärt bleibt jedoch die Stabilität der Ascorbinsäure im Endprodukt.

In dem Journal of Pharmaceutical Sciences, Vol. 57, Do 12, December 1968, Seiten 2119 - 2125 wird die Beständigkeit einer wäßrigen, 3 %igen und mit Kupferionen geimpften Ascorbinsäurelösung in Abhängigkeit von der Zugabe von Polysorbat 80 und der Temperatur untersucht.

Der Erfindung liegt demgegenüber die Aufgabe zugrunde, Ascorbinsäure in flüssiger, wasser- und fettlöslicher und in verkapselbarer Form (z.B. Gelatinekapsel) bereitzustellen, die über viele Monate hin beständig ist und in hoher Konzentration vorliegt.

Diese Aufgabe wird nach Erfindung mit einem Ascorbinsäure-Solubilisat, enthaltend Ascorbinsäure und einen Emulgator mit einem HLB-Wert von 9 - 18, gelöst, wobei der Ascorbinsäureanteil des Solubilisats bis zu etwa 20 Gew.-% beträgt, der Emulgator ein Polysorbat, vorzugsweise Polysorbat 80, mit einem Anteil von etwa 60 Gew.-% und der Rest ein Mischtocopherol und Wasser ist als Zusatz zu Lebensmitteln oder Kosmetika, Arzneimitteln und Nährlösungen zur Züchtung von Zell- und Bakterienkulturen sowie Algen, wobei die Ascorbinsäure in dem Ascorbinsäure-Solubulisat micelliert vorliegt und die Emulgatorhülle der Micellen die eingeschlossene Ascorbinsäure nur verzögert freigibt, so daß die antioxidative Wirkung des Ascorbinsäure-Solubulisats länger wirksam ist als bei Zusatz von einem Ascorbinsäurederivat wie Ascorbylpalmitat.

Eine wässrige Lösung von Ascorbinsäure, welche einen Überschuß an einem Emulgator mit einem HLB-Wert von 9 - 18 wie etwa ein Polysorbat, vorzugsweise Polysorbat 80 enthält, ist bei Raumtemperatur klar und fast gelartig, trübungsfrei mit Wasser verdünnbar und läßt sich bei Erwärmung auf ungefähr 35°C mit wässrigen oder fettigen Endprodukten aus dem Kosmetik- oder Lebensmittelbereich ohne weitere Verarbeitungsschritte mühelos homogen vermischen. Der Ascorbinsäureanteil der erfindungsgemäßen Lösung, der bis zu etwa 20 Gew% betragen kann, bleibt über wenigstens ein halbes Jahr praktisch verlustfrei erhalten. Der Polysorbatanteil beträgt zweckmäßig etwa 60 Gew%, der Rest ist Wasser. Das erfindungsgemäße Ascorbinsäuresolubilisat läßt sich leicht Kosmetika (Haut- und Haarpflegemitteln), Lebensmitteln, Arzneimitteln und Nährlösungen zur Züchtung von Zell- und Bakterienkulturen sowie von Algen (Mikroalgen) mit der Folge zusetzen, daß die Beständigkeit dieser Produkte wesentlich erhöht ist.

Die Ascorbinsäure liegt in dem Solubilisat micelliert vor. Eine elektronenmikroskopische Untersuchung eines 10%igen Ascorbinsäuresolubilisats in einer Verdünnung von 1:1000 zeigt einen Micellendurchmesser von etwa 100 nm. Da die Emulgatorhülle der Micellen die eingeschlossene Ascorbinsäure nur verzögert (retardierend) freigibt, bleibt die antioxydative Wirkung des erfindungsgemäßen Solubilisats in den erwähnten Mitteln in Kombination mit solubilisierten Misch-Tocopherolen länger wirksam als bei Zusatz etwa von Ascorbylpalmitat, also einem der eingangs genannten Ascorbinsäurederivate.

Ernährungsphysiologisch verhindert die Micellierung der Ascorbinsäure durch den Emulgator, daß bei oraler Gabe des erfindungsgemäßen Solubilisats die Ascorbinsäure bereits im mittleren Verdauungstrakt, also im Magen und Duodenum ihre Wirkung entfaltet und sich dabei verbraucht. Die micellierte Ascorbinsäure wird vielmehr erst im Dünndarm resorbiert.

Das Ascorbinsäuresolubilisat der Erfindung enthält einen Zusatz an einem Misch-Tocopherol. Durch Zusatz dieses Ascorbinsäure-Tocopherol-Solubilisats zu organischen Ölen, z.B. Pflanzenölen wie etwa Sonnenblumenöl, Distelöl, Leinöl und dergleichen kann deren Haltbarkeit ganz wesentlich verbessert werden. Als Tocopherol kommt besonders bevorzugt eine Mischung aus α-, β-, γ- und δ-Tocopherol in Betracht. Zu empfehlen ist ein Misch-Tocopherol, welches etwa 8,0 bis etwa 20,0 Gew% an α-Tocopherol, etwa 1,5 bis etwa 4,5 Gew% an β-Tocopherol, etwa 55,0 bis etwa 70,0 Gew% an γ-Tocopherol und etwa 15,0 bis etwa 27,0 Gew% an δ-Tocopherol enthält.

Wenn der erfindungsgemäßen Lösung eine Octadecatriensäure und/oder eine Octadecensäure, etwa in Form von α-Linolensäure, γ-Linolensäure, Linolsäure oder Ölsäure, zugesetzt ist, ist die Viskosität der Lösung herabgesetzt. Sie ist bei Raumtemperatur klar, zähflüssig, mit Wasser trübungsfrei verdünnbar bzw. mit wässrigen und/oder fettigen Nahrungsmitteln, Kosmetika und Pharmazeutika ohne weitere Verarbeitungsschritte vermischbar. Der Polysorbatgehalt der erfindungsgemäßen Lösung entfaltet eine Art Retardfunktion für die konservative Eigenschaft der Ascorbinsäure für die im allgemeinen leicht oxydierbaren Inhaltsstoffe von Salben und dergleichen Zubereitungen. Damit bleibt die erwünschte Schutzfunktion der Ascorbinsäure über einen längeren Zeitraum erhalten. Der Ascorbinsäuregehalt der Lösung kann zweckmäßig bei etwa 5 Gew% bis etwa 15 Gew% liegen. Die Lösung kann vorteilhaft etwa 10 Gew% bis etwa 20 Gew% an Octadecatriensäure und/oder Octadecensäure enthalten. Der Polysorbatanteil beträgt etwa 60 Gew%.

Wird der Wasseranteil der erfindungsgemäßen Lösung auf etwa 5 Gew% bis etwa 7 Gew% herabgesetzt und der Polysorbatanteil entsprechend erhöht, kann die bei Raumtemperatur zähflüssige Lösung auch Wirkstoffen beigemischt werden, die anschließend in Gelatinekapseln oder gelatinefreie Kapseln abgefüllt werden. Der geringe Wasseranteil der Lösung läßt die Kapselhülle unbeschädigt, wobei die Schutzfunktion der Ascorbinsäure für den Wirkstoff unbeeinträchtigt bleibt.

Erfindungsgemäß ist auch ein Verfahren zur Herstellung einer wässrigen Ascorbinsäurelösung gemäß Anspruch 6. Die in der Lösung sich bildenden Micellen mit einem Durchmesser von etwa 100 nm weisen eine doppelwandige Hülle aus radial orientierten Polysorbatmolekülen auf, wobei die Polysorbatmoleküle der Innenhülle mit ihren hydrophilen Abschnitten auf die Ascorbinsäurelösung ausgerichtet sind und die hydrophilen Abschnitte der Polysorbatmoleküle der Außenhülle sich außen anordnen. Die auf diese Weise gewonnene Lösung ist in Wasser trübungslos verdünnbar und läßt sich technisch ohne weitere Verarbeitungsschritte einsetzen.

Die Beständigkeit der Micellen wird erhöht, in dem der wässrigen Ascorbinsäurelösung ein im wesentlichen aus Triglyceriden bestehendes leichtes natürliches Öl, wie etwa Distelöl, zusammen mit dem Emulgator zugesetzt wird. Die sich dabei bildenden Ölmicellen adhärieren an den Ascorbinsäuremicellen und schützen diese.

Zweckmäßig wird der Lösung nach leichtem Erwärmen auf etwa 50°C unter Rühren eine Octadecatriensäure und/oder eine Octadecensäure etwa in Form von α-Linolensäure, γ-Linolensäure, Linolsäure oder Ölsäure vor der Zugabe von Polysorbat zugesetzt. Für die Befüllung von Kapseln, deren Hülle aus Gelatine bestehen oder die gelatinefrei sein kann, ist es vorteilhaft, wenn Ascorbinsäure in der gleichen Menge aqua dest gelöst, der Lösung die gleiche bis maximal doppelte Menge wie Ascorbinsäure an Fettsäuren unter leichter Erwärmung zugegeben und die etwa zwei- bis etwa dreifache Menge an Polysorbat 80 zugesetzt und das Gemisch auf etwa 80°C erwärmt und gerührt wird.

Erfindungsgemäß ist auch eine Mischung, ein Mischsolubilisat, ein Verfahren und eine Verwendung gemäß der Ansprüche 7-10.

Die nachstehenden Ausführungsbeispiele verdeutlichen die Erfindung.

### Beispiel 1 (nicht erfindungsgemäß)

20g Ascorbinsäure werden in 20g destilliertem, entgasten Wasser vollständig aufgelöst. Der Lösungsvorgang kann beschleunigt werden, wenn das Wasser auf etwa 45 °C erwärmt wird. Anschließend werden der Lösung 60g Polysorbat 80 unter Rühren und Aufwärmen auf etwa 80°C zugegeben. Es wird gerührt bis zur Klarheit und Homogenität des Solubilisats, das bei Raumtemperatur klar und fast gelartig erscheint, in Wasser trübungslos verdünnbar ist und sich ohne weitere Verarbeitungsschritte den zu konservierenden Zusammensetzungen oder Nahrungsmitteln, Getränken, Kosmetika und Pharmazeutika zusetzen läßt.

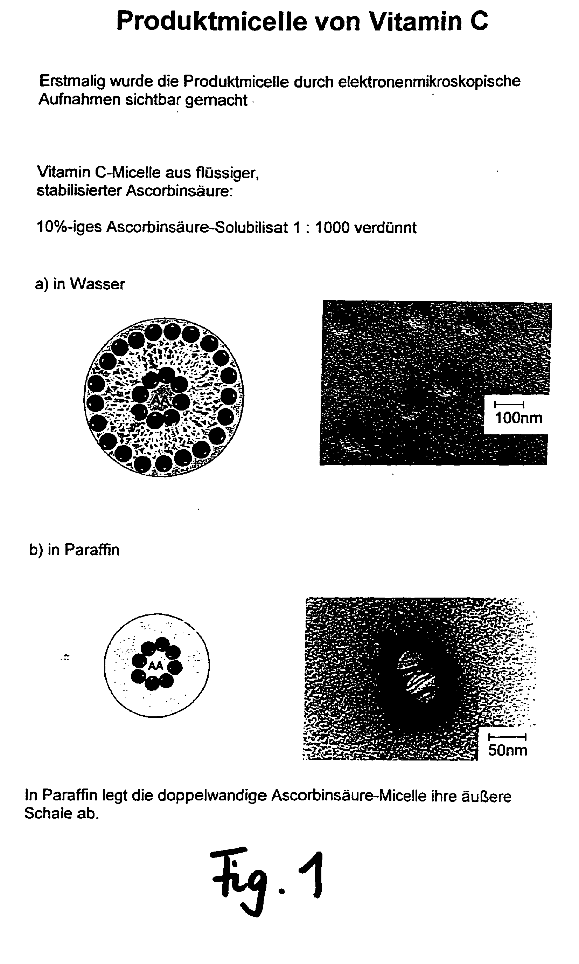

Für ein geringer konzentriertes Solubilisat löst man 10g Ascorbinsäure in 10g aqua dest auf und gibt 80g Polysorbat 80 hinzu, verfährt im übrigen wie vorstehend. Von dem daraus erhaltenen 10%igen Ascorbinsäuresolubilisat wurde nach dessen wässriger Verdünnung zu 1 : 1000 eine elektronenmikroskopische Aufnahme gemacht, die in Figur 1 wiedergegeben ist. Man sieht, daß die Ascorbinsäuresolubilisatmicellen einen Durchmesser von etwa 100 nm haben. Einbringen des Solubilisats in Paraffin läßt den Micellendurchmesser auf die Hälfte schrumpfen, wie man aus der unteren elektronenmikroskopischen Aufnahme der Figur 1 erkennt. Diese Erscheinung kann man damit erklären, daß die Micellen in Paraffin die äußere Polysorbathülle verlieren.

### Beispiel 2

Wie bei Beispiel 1 löst man 10 Gew% Ascorbinsäure (bezogen auf die Gesamtmenge an Ascorbinsäuresolubilisat = 100%) in 10Gew% aqua dest und gibt zu dieser wässrigen Lösung 70 Gew% Polysorbat 80 zusammen mit 10 Gew% eines leichten Pflanzenöls wie etwa Distelöl oder Leinöl. Man erwärmt auf mindestens 60°C und rührt bis zur Klarheit und Homogenität des Solubilisats, das sich bei Raumtemperatur in Wasser klar und rückstandsfrei lösen läßt.

Reduziert man den Gehalt an Ascorbinsäure auf etwa 7 Gew% und den Wassergehalt auf ebenfalls etwa 7 Gew% und erhöht die weiteren Anteile des Solubilisats entsprechend, kann es wegen des relativ geringen Wasseranteils besonders gut als Konservierungsmittel für Wirkstoffe eingesetzt werden, die als Retardpräparate verkapselt werden sollen. Die Kapselhülle wird von dem Solubilisat praktisch nicht angegriffen, was bei einem höheren Wassergehalt zu befürchten wäre.

### Beispiel 3

10 g Ascorbinsäure werden in 10 g aqua dest. aufgelöst und der Lösung 20 g Distelöl zugegeben. Dieser Mischung werden 110 g Polysorbat 80 zugesetzt, das Ganze unter Rühren auf etwa 100°C bis zur Wasserfreiheit, d.h. bis zur Beendigung des Kochens, erhitzt. Nach Abkühlen auf Zimmertemperatur liegt ein 6,5 %iges Ascorbinsäure-Solubilat in dem Lösungsvermittler vor, dessen Wassergehalt deutlich unter 5 Vol% liegt und das sowohl in Wasser als auch in Fetten und Ölen löslich ist. Eine Prüfung des Ascorbinsäuregehalts der Lösung durch ein unabhängiges Chemisches Untersuchungslabor zum Zeitpunkt der Herstellung der Lösung und mehr als fünf Monate später ergab einen Ascorbinsäureverlust während dieser Zeit von nur etwa 3%.

### Beispiel 4

Man geht aus von dem Solubilisat nach Beispiel 2. Diesem wird ein Misch-Tocopherol-Solubilisat zugegeben, das auf folgende Weise gewonnen wird: 10 Gew% Misch-Tocopherol (bezogen auf das Misch-Tocopherol-Solubilisat = 100Gew%) wird mit 90 Gew% Polysorbat 20 durch Rühren vermischt, wobei der Mischvorgang durch Erwärmen auf etwa 60°C beschleunigt wird. Das Rühren wird solange fortgesetzt, bis Klarheit erreicht ist und das Solubilisat sich in Wasser leicht lösen läßt. Für das Misch-Tocopherol empfiehlt sich eine Zusammensetzung von 91 mg/g Misch-Tocopherol an α-Tocopherol, 21 mg/g an β-Tocopherol, 608 mg/g an γ-Tocopherol und 209 mg/g an δ-Tocopherol.

Sodann werden etwa 3 Gewichtsteile des Solubilisats nach Beispiel 2 erste Alternative mit etwa 7 Gewichtsteilen des Misch-Tocopherol-Solubilisats vermischt, wobei eine leichte Erwärmung auf etwa 50°C den Mischvorgang beschleunigt. Das Rühren in der Wärme wird solange ausgeführt, bis sich ein homogenes und klares Solubilisat ergeben hat. 1g dieses Produktes enthält somit etwa 70 mg Misch-Tocopherol und etwa 30 mg Ascorbinsäure. Dieses Solubilisat kann als wirksames Antioxydans zur Verbesserung der Haltbarkeit von Lebensmittel-Farbstoffen, Ölen, Kosmetika, Pharmazeutika und dergleichen eingesetzt werden.

Vergleichstests belegen, daß sich dieses Solubilisat unabhängig von den Eigenschaften des Endproduktes (hydrophil oder hydrophob) direkt und ohne Produktionszwischenschritte in diese einarbeiten läßt und einen besseren Schutz vor Oxydation bietet als die gleiche Menge an Ascorbinsäure aus einem Ascorbinsäurederivat wie beispielsweise Ascorbylpalmitat. Auch die relative Farblosigkeit des Solubilisats im Vergleich zu den Ascorbinsäurederivaten stellt einen Vorteil dar. Je nach Bedarf können beispielsweise 1 bis 10 g des Solubilisats etwa 1000 g des Endproduktes zugegeben werden.

Der ernährungsphysiologische Vorteil des Solubilisats liegt in den magensäurestabilen Micellen, die dafür sorgen, daß Ascorbinsäure (Vitamin C) und (bei Verwendung von α-Tocopherol) Vitamin E den Dünndarm verlustfrei erreichen, um dort resorbiert werden zu können. Dieser Vorteil läßt sich auch für weitere Vitamine, beispielsweise Retinol (Vitamin A) und β-Carotin nutzen und mit folgendem beispielshaften Multivitaminpräparat erreichen:

### Beispiel 5

20 Gew% α-Tocopherol werden, gegebenenfalls in der Wärme von etwa 50°C, mit 80 Gew% Polysorbat 20 bis zur Klarheit und Homogenität durch Rühren vermischt.
10 Gew% Retinol werden auf die gleiche Weise mit 90 Gew% Polysorbat 80 vermischt.
Schließlich werden 10 Gew% eines Konzentrats, das etwa 30% β-Carotin enthält (erhältlich bei La Roche), mit 90 Gew% Polysorbat 80 in entsprechender Weise zu einem rotbraunen, transparenten Solubilisat vermischt, das sich in temperiertem Wasser klar lösen läßt.
Sodann werden 85 Gew% des Ascorbinsäuresolubilisats nach Beispiels 2 erste Alternative mit 10 Gew% vorstehenden α-Tocopherolsolubilisats sowie mit 2 Gew% des vorstehenden Retinolsolubilisats und schließlich mit 3 Gew% des β-Carotinsolubilisats zweckmäßig in leichter Wärme durch Rühren miteinander vermischt, bis ein homogenes und transparentes Mischsolubilisat vorliegt. Dieses wasserlösliche Vitaminsolubilisat läßt sich in Gelatine- oder gelatinefreien Kapseln verpacken oder in wässrigen und/oder fettlöslichen Endprodukten ohne zusätzliche Verarbeitungsschritte direkt einarbeiten.

Eingebracht in Wasser und/oder klaren Frucht- oder Obstsäften ergibt das erfindungsgemäße Vitaminsolubilisat im Gegensatz zu Emulsionen oder Liposomen eine stabile und klare Lösung. Die Produktmicellen sind magensäurestabil. Die Resorption der in den Micellen befindlichen fettlöslichen Substanzen wie Vitamin A, Vitamin E und β-Carotin erfolgt im Dünndarm ohne Beteiligung von Gallensalzen und Enzymen. Daher sind die genannten Wirkstoffe in der vorliegenden micellierten Form schneller bioverfügbar.

1 Gramm des Vitaminsolubilisats der vorstehenden Zusammensetzung eingearbeitet in Lebensmittel oder eingebracht in Kapseln deckt den menschlichen Tagesbedarf an den Vitaminen A, C, E und β-Carotin. Auf diesen Umstand ist die vorstehend angegebene zahlenmäßige Zusammensetzung des Vitaminsolubilisats abgestellt. Darin kommt zum Ausdruck, daß der Tagesbedarf an Vitamin C wesentlich höher ist als derjenige an anderen Vitaminen. Es liegt jedoch im Rahmen der Erfindung, für das Vitaminsolubilisat auch andere Zusammensetzungen zu wählen und/oder auf das eine oder andere Vitamin überhaupt ganz zu verzichten, wenn der vorgesehene Verwendungszweck das Vorhandensein eines bestimmten Vitamins im Vitaminsolubilisat nicht erforderlich oder wünschenswert macht.

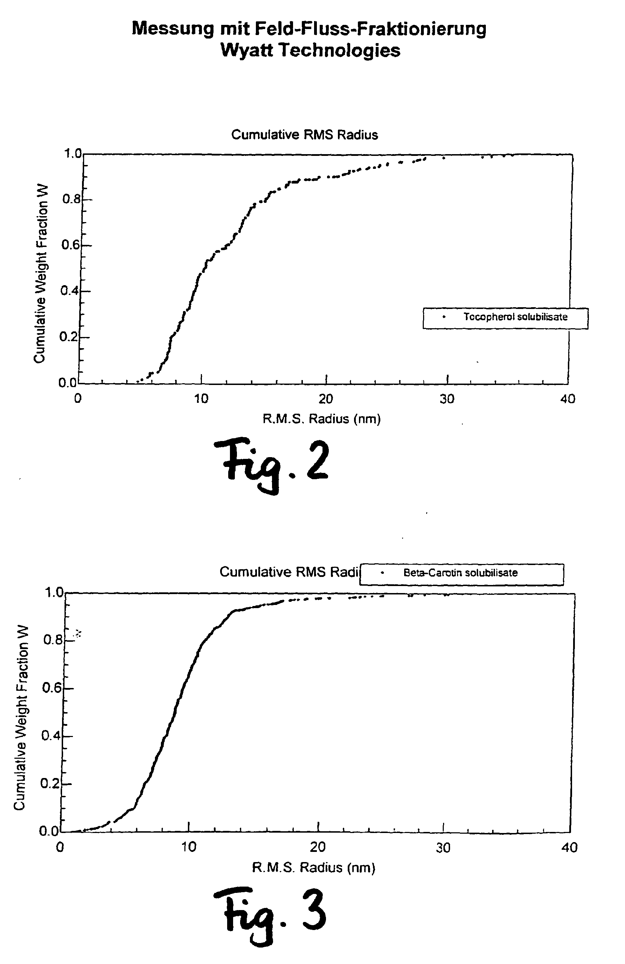

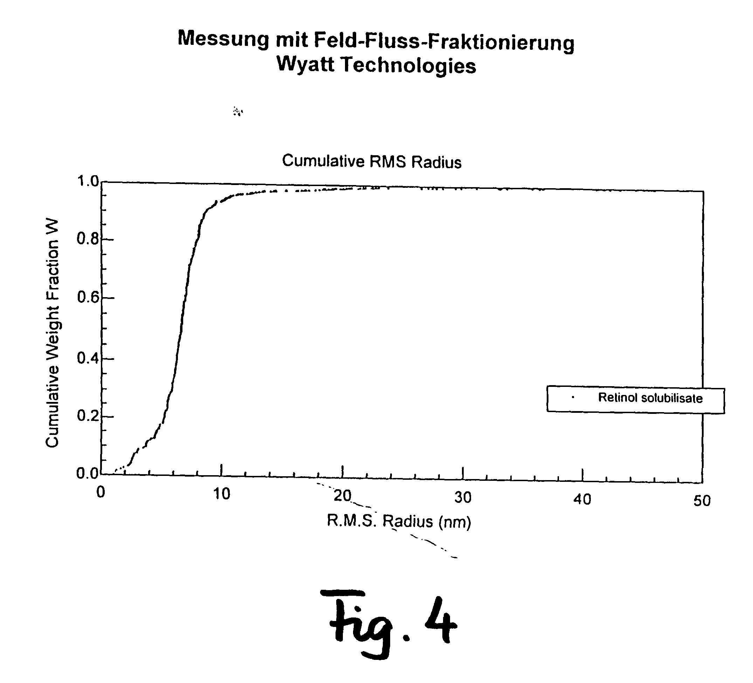

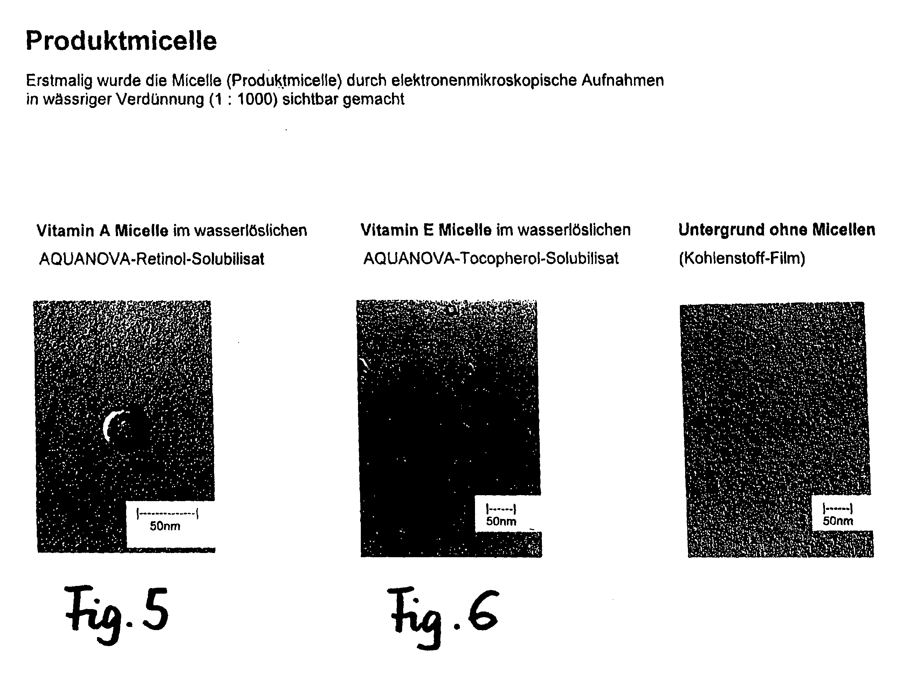

Die Figuren 2, 3 und 4 zeigen die mittleren Micellenradien des α-Tocopherol-solubilisats, des β-Carotinsolubilisats und des Retinolsolubilisats. Wie ersichtlich liegen die mittleren Micellenradien bei 10 nm und für Retinolsolubilisat sogar bei nur etwa 8 nm. Die Messungen wurden mit der Feld-Fluß-Fraktionierung der Wyatt Technologies durchgeführt. Figur 5 zeigt eine elektronenmikroskopische Aufnahme einer Vitamin A Micelle, und Figur 6 eine elektrononmikroslopische Aufnahme von Vitamin E Micellen; die Proben waren eine wässrige Verdünnung von 1 : 1000 der oben beschriebenen Solubilisate.

## Patentansprüche

1. Ascorbinsäure-Solubilisat enthaltend Ascorbinsäure und einen Emulgator mit einem HLB-Wert von 9 - 18, wobei der Ascorbinsäureanteil des Solubilisats bis zu etwa 20 Gew.-% beträgt, der Emulgator ein Polysorbat, vorzugsweise Polysorbat 80, mit einem Anteil von etwa 60 Gew.-% und der Rest ein Mischtocopherol und Wasser ist als Zusatz zu Lebensmitteln oder Kosmetika, Arzneimitteln und Nährlösungen zur Züchtung von Zell- und Bakterienkulturen sowie Algen,
wobei die Ascorbinsäure in dem Ascorbinsäure-Solubulisat micelliert vorliegt und die Emulgatorhülle der Micellen die eingeschlossene Ascorbinsäure nur verzögert freigibt, so daß die antioxidative Wirkung des Ascorbinsäure-Solubulisats länger wirksam ist als bei Zusatz von einem Ascorbinsäurederivat wie Ascorbylpalmitat.

2. Solubilisat nach Anspruch 1, bei dem das Mischtocopherol etwa 8,0 bis etwa 20,0 Gew.-% an α-Tocopherol, etwa 1,5 bis etwa 4,5 Gew.-% an β-Tocopherol, etwa 55,0 bis etwa 70,0 Gew.-% an γ-Tocopherol und etwa 15,0 bis etwa 27,0 Gew.-% an δ-Tocopherol enthalt.

3. Solubilisat nach einem der vorstehenden Ansprüche mit einem Gehalt an Octadecatriensäure.

4. Solubilisat nach Anspruch 3 mit einem Gehalt von etwa 10 Gew.-% bis etwa 20 Gew.-% an Octadecatriensäure.

5. Solubilisat nach einem der Ansprüche 1 bis 4 enthaltend γ-Linolensäure, Linolsäure oder Ölsäure.

6. Verfahren zur Herstellung eines Ascorbinsäures-Solubilisats, bei dem 10 Gew.-% Ascorbinsäure in 10 Gew.-% aqua dest aufgelöst und dieser wässrigen Lösung 70 Gew.-% Polysorbat 80 zusammen mit 10 Gew.-% eines leichten Pflanzenöls wie etwa Distelöl oder Leinöl zugegeben werden, und anschließend auf mindestens 60°C erwärmt und bis zur Klarheit und Homogenität gerührt wird.

7. Mischung bestehend aus 3 Gewichtsteilen des nach Anspruch 6 hergestellten Ascorbinsäure-Solubilisats und 7 Gewichtsteilen eines Misch-Tocopherol-Solubilisats, wobei das Misch-Tocopherol-Solubilisat aus 10 Gew.-% Misch-Tocopherol und 90 Gew.-% Polysorbat 20, jeweils bezogen auf 100 Gew.-% Misch-Tocopherol-Solubilisat, besteht.

8. Mischsolubilisat bestehend aus 85 Gew.-% des nach Anspruch 6 hergestellten Ascorbinsäure-Solubilisats, 10 Gew.-% eines α-Tocopherol-Solubilisats, 2 Gew.-% eines Retinolsolubilisats und 3 Gew.-% eines β-Carotinsolubilisats, bezogen auf die Mischung 100 Gew.-%, wobei das α-Tocopherol-Solubilisat aus 20 Gew%.-% α-Tocopherol und 80 Gew.-% Polysorbat 20, bezogen auf α-Tocopherol-Solubilisat gleich 100 Gew.-%, besteht, das Retinolsolubilisat aus 10 Gew.-% Retinol und 90 Gew.-% Polysorbat 80, bezogen auf 100 Gew.-% Retinolsolubilisat besteht, und das β-Carotinsolubilisat aus 10 Gew.-% eines Konzentrats, das etwa 30 % β-Carotin enthalt, und 90 Gew.-% Polysorbat 80 besteht, wobei sich die für das β-Carotinsolubilisat angegebenen Prozentzahlen auf das β-Carotinsolubilisat = 100 Gew.-% beziehen.

9. Verfahren zur Herstellung eines Ascorbinsäure-Solubilisats, bei dem 10 Gewichtsteile Ascorbinsäure in 10 Gewichtsteilen aqua dest gelöst und der Lösung 20 Gewichtsteile Distelöl zugegeben und der Mischung 110 Gewichtsteile Polysorbat 80 zugesetzt werden, wonach das Ganze unter Rühren auf etwa 100°C bis zur Beendigung des Kochens erhitzt wird, so dass nach Abkühlen auf Zimmertemperatur ein 6,5%iges Ascorbinsäure-Solubilisat in dem Lösungsvermittler vorliegt, dessen wassergehalt deutlich unter 5 Vol% liegt.

10. Verwendung des Mischsolubilisats nach Anspruch 8 als Füllung von Kapseln oder als Zusatz zu klaren Fruchtsäften oder Obstsäften.

## Claims

1. Solubilizate of ascorbic acid containing ascorbic acid and an emulsifier with an HLB-value of 9 - 18, wherein the ascorbic acid proportion of the solubilizate amounts up to about 20% by weight, the emulsifier is a polysorbate, preferably polysorbate 80, having a proportion of about 60% by weight and the remainder is a mixed tocopherol and water as an additive to foodstuffs or cosmetics, medicaments and nutrient solutions for growth of cell and bacterial cultures and algae, wherein the ascorbic acid is present in micelle-form in the solubilizate of ascorbic acid and the emulsifier casing of the micelles releases the encased ascorbic acid only in a retarded manner, so that the anti-oxidative effect of the solubilizate of ascorbic acid is effective for longer than with the addition of an ascorbic acid derivative such as ascorbyl palmitate.

2. Solubilizate as claimed in claim 1, wherein the mixed tocopherol contains about 8.0 to about 20.0 % by weight of α-tocopherol, about 1.5 to about 4.5 % by weight of β-tocopherol, about 55.0 to about 70.0 % by weight of γ-tocopherol and about 15.0 to about 27.0 % by weight of δ-tocopherol.

3. Solubilizate as claimed in any one of the preceding claims having a content of octadecatrienoic acid.

4. Solubilizate as claimed in claim 3, having a content of about 10 % by weight to about 20 % by weight of octadecatrienoic acid.

5. Solubilizate as claimed in any one of claims 1 to 4, containing γ-linolenic acid, linoleic acid or oleic acid.

6. Method of preparing a solubilizate of ascorbic acid, wherein 10 % by weight of ascorbic acid is dissolved in 10 % by weight of distilled water and 70 % by weight of polysorbate 80 together with 10 % by weight of a light vegetable oil such as safflower oil or linseed oil are added to this aqueous solution, with subsequent heating to at least 60°C and stirring to the point of clarity and homogeneity.

7. Mixture consisting of 3 parts by weight of the solubilizate of ascorbic acid prepared in accordance with claim 6, and of 7 parts by weight of a mixed tocopherol solubilizate, wherein the mixed tocopherol solubilizate consists of 10 % by weight of mixed tocopherol and 90 % by weight of polysorbate 20, in each case based upon 100 % by weight of mixed tocopherol solubilizate.

8. Mixed solubilizate consisting of 85 % by weight of the solubilizate of ascorbic acid prepared in accordance with claim 6, of 10 % by weight of an α-tocopherol solubilizate, 2 % by weight of a retinol solubilizate and 3 % by weight of a β-carotene solubilizate, based upon the 100 % by weight mixture, wherein the α-tocopherol solubilizate consists of 20 % by weight of α-tocopherol and 80 % by weight of polysorbate 20, based upon α-tocopherol solubilizate equal to 100 % by weight, the retinol solubilizate consists of 10 % by weight of retinol and 90 % by weight of polysorbate 80, based upon 100 % by weight of retinol solubilizate, and the β-carotene solubilizate consists of 10 % by weight of a concentrate, which contains about 30 % of β-carotene, and of 90 % by weight of polysorbate 80, wherein the percentages stated for the β-carotene solubilizate relate to the β-carotene solubilizate = 100 % by weight.

9. Method of preparing a solubilizate of ascorbic acid, wherein 10 parts by weight of ascorbic acid are dissolved in 10 parts by weight of distilled water and 20 parts by weight of safflower oil are added to the solution and 110 parts by weight of polysorbate 80 are added to the mixture, after which the entire mixture is heated with stirring to about 100°C up to completion of boiling, so that after cooling to room temperature a 6.5 % solubilizate of ascorbic acid is present in the solubiliser, the water content of which is considerably less than 5 % by volume.

10. Use of the mixed solubilizate as claimed in claim 8 as a filling of capsules or as an additive to clear fruit juices.

## Revendications

1. Produit de solubilisation d'acide ascorbique contenant de l'acide ascorbique et un émulsifiant, ayant une valeur d'équilibre hydrophile-lipophile de 9 - 18, la fraction d'acide ascorbique du produit de solubilisation s'élevant jusqu'à environ 20 % en poids, l'émulsifiant étant un polysorbate, avantageusement le Polysorbate-80 en une proportion d'environ 60 % en poids et le reste étant formé d'eau et d'un mélange de tocophérols , en tant qu'additif pour produits alimentaires ou cosmétiques, médicaments et solutions nutritives pour la culture de cellules et pour cultures de bactéries ainsi que d'algues, l'acide ascorbique étant présent à l'état de micelles dans le produit de solubilisation d'acide ascorbique et l'enveloppe d'émulsifiant des micelles ne libérant qu'avec un effet retardé l'acide ascorbique inclus , de sorte que l'action antioxydante du produit de solubilisation de l'acide ascorbique a une plus longue durée d'efficacité que lors de l'addition d'un dérivé d'acide ascorbique comme le palmitate d'ascorbyle.

2. Produit de solubilisation suivant la revendication 1, dans lequel le mélange de tocophérols contient environ 8,0 à environ 20,0 % en poids d'α-tocophérol, environ 1,5 à environ 4,5 % en poids de β-tocophérol, envron 55,0 à environ 70,0 % en poids de γ-tocophérol et environ 15,0 à environ 27,0 % en poids de δ-tocophérol.

3. Produit de solubilisation suivant l'une des revendications précédentes, ayant une teneur en acide octadécatriénoïque.

4. Produit de solubilisation suivant la revendication 3, ayant une teneur d'environ 10 % en poids à environ 20 % en poids d'acide octadécatriénoïque.

5. Produit de solubilisation suivant l'une des revendications 1 à 4, contenant de l'acide γ-linolénique, de l'acide linolique ou de l'acide oléique.

6. Procédé de préparation d'un produit de solubilisation d'acide ascorbique, dans lequel 10 % en poids d'acide ascorbique sont dissous dans 10 % en poids d'eau distillée et cette solution aqueuse est additionnée de 70 % en poids de Polysorbate-80 conjointement avec 10 % en poids d'une huile végétale légère telle que, par excemple, de l'huile de chardon ou de l'huile de lin, puis chauffée à 60°C au moins et agitée jusqu'à ce qu'elle soit claire et homogène.

7. Mélange constitué de 3 parties en poids du produit de solubilisation d'acide ascorbique préparé selon la revendication 6 et de 7 parties en poids d'un produit de solubilisation d'un mélange de tocophérols, le produit de solubilisation du mélange de tocophérols étant constitué de 10 % en poids de mélange de tocophérols et de 90 % en poids de Polysorbate-80, dans chaque cas pour 100 parties en poids de produit de solubilisation du mélange de tocophérols.

8. Produit de solubilisation en mélange constitué de 85 % en poids du produit de solubilisation d'acide ascorbique préparé selon la revendication 6, 10 % en poids d'un produit de solubilisation d'α-tocophérol, 2 % en poids d'un produit de solubilisation de rétinol et 3 % en poids d'un produit de solubilisation de β-carotène, par rapport à 100 % en poids de mélange, le produit de solubilisation d'α-tocophérol étant constitué de 20 % en poids d'α-tocophérol et de 80 % en poids de Polysorbate-20, par rapport au produit de solubilisation de l'α-tocophérol égalant 100 % en poids, le produit de solubilisation de rétinol étant constitué de 10 % en poids de rétinol et de 90 % en poids de Polysorbate-80, par rapport à 100 % en poids de produit de solubilisation de rétinol, et le produit de solubilisation de β-carotène étant constitué de 10 % en poids d'un concentré qui contient environ 30 % de β-carotène, et de 90 % en poids de Polysorbate-80, les pourcentages indiqués pour le produit de solubilisation du β-carotène se rapportant au produit de solubilisation du β-carotène égalant 100 % en poids.

9. Procédé de préparation d'un produit de solubilisation d'acide ascorbique, dans lequel 10 parties en poids d'acide ascorbique sont dissoutes dans 10 parties en poids d'eau distillée et cette solution est additionnée de 20 parties en poids d'huile de chardon, et le mélange est additionné de 110 parties en poids de Polysorbate-80, puis le tout est chauffé sous agitation à environ 100°C jusqu'à la fin de l'ébullition, de manière à obtenir après refroidissement à la température ambiante un produit de solubilisation d'acide ascorbique à 6,5 % dans le solvant auxiliaire, dont la teneur en eau est nettement inférieure à 5 % en volume.

10. Utilisation du produit de solubilisation en mélange selon la revendication 8 comme charge de capsules ou comme additif pour des jus de fruits clairs et des jus de fruits pressés.
